Europäisches Patentamt

European Patent Office

Office européen des brevets

(11) Publication number: 0 313 411
A1

# EUROPEAN PATENT APPLICATION

(21) Application number: 88309968.1

(22) Date of filing: 21.10.88

(51) Int. Cl.⁴: G 01 N 33/48

(30) Priority: 23.10.87 GB 8724845

(43) Date of publication of application:
26.04.89 Bulletin 89/17

(84) Designated Contracting States:
AT BE CH DE ES FR GB GR IT LI LU NL SE

(71) Applicant: MB GROUP PLC (formerly known as Metal
Box plc)
Caversham Bridge House Waterman Place
Reading RG1 8DN Berkshire (GB)

(72) Inventor: Ackland, Martin Robert
Cedar Cottage East Hendred
Wantage Oxfordshire OX12 8RT (GB)

Murphy, Thomas Patrick
5 Kelly's Road
Wheatley Oxfordshire OX9 1NT (GB)

(74) Representative: Sawers, Lawrence Peter et al
PAGE, WHITE & FARRER 5 Plough Place New Fetter Lane
London EC4A 1HY (GB)

(54) Methods of detecting micro-organisms.

(57) In an electrochemical method of detecting micro-organ-
isms in a sample, an electrical DC input is applied across a
working electrode (12) and a secondary electrode (13) in a cell
(10) containing the sample (11) while maintaining constant
current or constant potential conditions in the cell, and
monitoring either potential conditions or current, respectively.
A sharpe change in the monitored condition indicates that
growth of micro-organisms has reached a particular stage. The
potential of the working electrode (12) with respect to an
adjacent reference electrode (14) or with respect to the
secondary electrode (13) may be maintained constant and the
current monitored. Preferably the current is maintained con-
stant and either the voltage across the cell or the potential of
the working electrode (12) with reference to an adjacent
reference electrode (25) is monitored. A potential control
amplifier (15) may be used to control the current or potential
conditions.

FIG.1.

**Description**

## METHODS OF DETECTING MICRO-ORGANISMS

This invention relates to methods of detecting micro-organisms in samples of a substance by electro-chemical means.

It has been conventional practice to test a substance for the presence of micro-organisms by injecting a sample of the substance into a growth medium and monitoring the sample visually or photometrically for the development of turbidity. It is also known that the growth of micro-organisms in such samples can be detected by measuring the changing potential between electrodes in contact with the sample. It has been observed that, when the growth of the micro-organisms reaches a particular stage, the potential shows a marked change, e.g. a steep fall.

In our British Patent Specification No. 2142433 we have described apparatus by which the growth of micro-organisms in a plurality of samples can be monitored electrochemically, by monitoring the potential difference developed between two electrodes of different metals, e.g. gold and aluminium, in contact with the samples.

According to the present invention, a method of detecting micro-organisms in a sample of a substance comprises the steps of placing the sample in a cell so as to be in contact with at least two electrodes, one of which is a working electrode and another a secondary electrode, applying an electrical D.C. input across said working and secondary electrodes while maintaining constant current or constant potential conditions in the cell, and monitoring either potential conditions or current, respectively, in the cell against time.

A marked change in the potential conditions or current indicates that micro-organisms were present in the sample and that their growth has now reached a particular stage. The time taken to reach this stage is an indication of the number of micro-organisms originally present.

The use of an applied electrical input in accordance with the invention removes the need to choose the electrode materials for the potential difference which they can develop galvanically and so enables one to choose the materials for their corrosion resistance, compatibility with the micro-organisms and other desirable characteristics. Thus the two electrodes may both be of a noble metal such as platinum.

Furthermore, it is possible by appropriate choice of the applied potential to increase the sensitivity of the detection by shifting the starting conditions nearer to the point at which a marked change will occur if micro-organisms are present.

In a first form of the invention, the potential of a third, reference, electrode disposed adjacent to the working electrode is maintained constant with respect to that of the working electrode, and the current flowing through the cell is monitored against time.

In a second form of the invention, the potential of the secondary electrode with respect to the working electrode is maintained constant so that the voltage across the cell is kept constant, and the current flowing through the cell is monitored against time.

In a third form of the invention, the current flowing in the cell is maintained constant, and the voltage across the cell is monitored against time.

In a fourth form of the invention, the current flowing in the cell is maintained constant and the potential of the working electrode with respect to a third, reference, electrode, disposed adjacent to the working electrode, is monitored.

In either of the two latter forms of the invention, conveniently a potential difference is established across a fixed resistance between the working electrode and the adjacent side of the DC input and is maintained constant in order to maintain the current constant.

The constant current or potential conditions may be established and maintained by means of a potential control amplifier (as herein defined) whose output terminals are connected across the secondary and working electrodes of the cell to form the DC input.

The term "potential control amplifier" is used herein to indicate a kind of operational amplifier which is arranged to receive one input signal from an "internal" constant potential source and another input at a "reference" terminal from an external element whose potential is to be kept at the same level as the constant potential source, the amplifier producing a D.C. output signal at an output terminal which is dependent upon any difference between the internal and external inputs and is fed back to adjust the potential of the external element to equal that of the internal source. The other side of the constant potential source is connected to a second output terminal of the potential control amplifier, so that the reference terminal and the external element connected thereto are maintained at a constant potential with respect to the second output terminal.

For maintaining a constant current through the cell, the working electrode may be connected to the respective output terminal of the potential control amplifier through a fixed resistance and the reference terminal connected to the same side of the fixed resistance as the working electrode, so that the potential difference across the fixed resistance, and consequently the current drawn through the resistance and the cell, is kept constant.

In general, it is more convenient to operate under galvanostatic conditions, i.e. with a constant current flowing through the sample.

Specific embodiments of the invention will now be described by way of example and with reference to the accompanying drawing, in which:-

Figure 1 is a diagrammatic illustration of an apparatus for detecting micro-organisms, operating in accordance with the first form of the invention mentioned above,

Figure 2 is an illustration of a similar apparatus operating in accordance with the second form of the invention,

Figure 3 illustrates a similar apparatus arranged to operate in accordance with the third form of the invention, and

Figure 4 illustrates a similar apparatus operating in accordance with the fourth form described above.

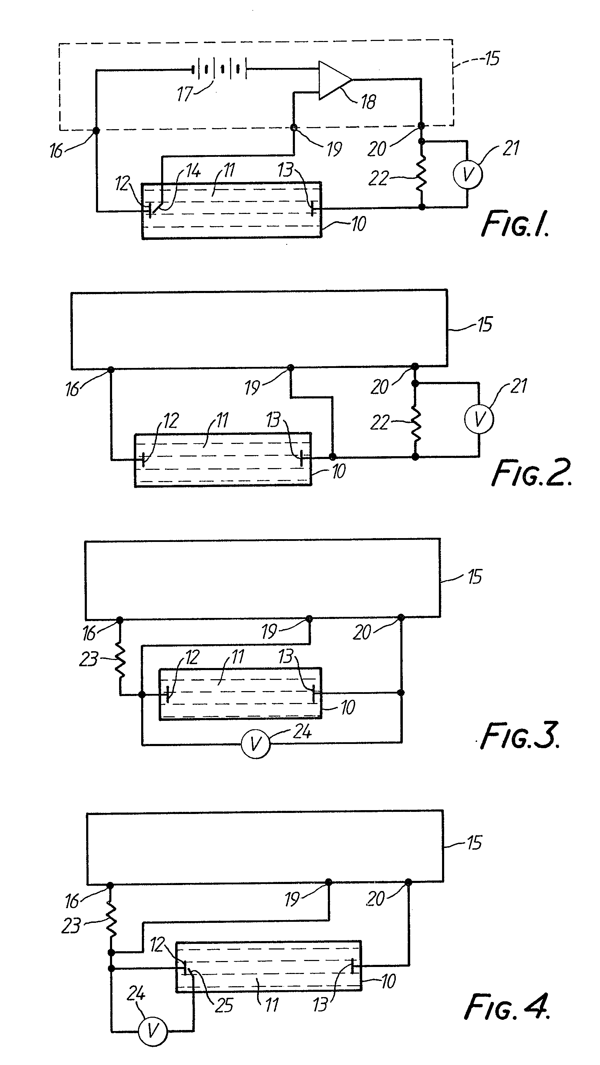

In the embodiment illustrated in Figure 1, a sample of the substance to be tested is placed in a cell 10, e.g. by injection into a growth medium such as a nutrient broth 11. The cell 10 is provided with a working electrode 12, a secondary electrode 13 and a third, reference, electrode 14 disposed adjacent to the working electrode.

The electrodes 12,13,14 are shown as connected to a potential control amplifier or potentiostat of known type as described above and generally referenced as 15, for detection of a micro-organism. Electrode 12 is the working electrode and is accordingly connected to the "working" output terminal 16 of the potentiostat 15. The potentiostat 15 includes an "internal" constant potential source 17 shown conventionally as a battery) one side of which is connected to the working terminal 16 and the other side of which is connected to one input of an operational amplifier 18 whose other input is connected via the "reference" terminal 19 to the reference electrode 14. The output of the amplifier is connected via the output terminal 20 and resistance 22 to the secondary electrode 13. A voltmeter 21 is connected across resistance 22 to measure the current flowing through the cell 10. The potentiostat 15 reacts to any inequality between the potentials of the source 17 and of the reference terminal 19, with respect to the working terminal 16, by varying the voltage at the output terminal 20 so as to alter the current through the cell 10 to the extent needed to restore the potential of the reference terminal 19 and electrode 14 with respect to the working terminal 16 and electrode 12 to the pre-set value.

If the sample injected into the cell 10 contains micro-organisms, they will grow in the growth medium 11. When the growth reaches a particular stage, commonly at about 1,000,000 organisms per millilitre, a sharp change will be noted in the current flowing through the cell 10 between the electrodes 12, 13. The time taken to reach this stage is a measure of the initial concentration of micro-organisms in the sample. The reason for the sharp change in current is not definitely known, but it is believed to be due to the concentration of dissolved oxygen, or of another species which is consumed by the micro-organisms in competition with the electro-chemical process, being reduced below a critical value.

Figure 2 illustrates an alternative embodiment of the invention in which the separate reference electrode 14 is omitted, being in effect combined with the secondary electrode 13. The reference terminal 19 is thus connected to the secondary electrode 13 so that the potential of the secondary electrode 13 with respect to the working electrode 12, and hence the voltage across the cell is kept constant. In other respects, the apparatus works in the same way as that of Figure 1 and the current flowing through the cell 10 is monitored by the voltmeter 21 as before.

Figure 3 illustrates how the apparatus can be arranged to operate galvanostatically. The arrangement is similar to that of Figure 2, except that the reference terminal 19 is connected to the working electrode or cathode 12 and a fixed resistance 23 is interposed between the cathode 12 and the working terminal 16. The potential difference across the resistance 23 is thus maintained constant, so that the current drawn through the resistance 23 and the cell 10 remains constant. The voltmeter 21 and resistance 22 are accordingly omitted and replaced by a voltmeter 24 connected across the electrodes 12,13 to monitor the voltage across the cell.

In this case, the sharp change in electrical conditions in the cell which takes place when the micro-organisms have reached a particular concentration is reflected by a sharp change in the voltage across the cell as the potentiostat reacts to maintain the current constant.

In the embodiment of Figure 4 the apparatus again operates galvanostatically. The connections are similar to those of Figure 3 except that a separate reference electrode 25 is provided adjacent to the working electrode 12 and connected to the working electrode via voltmeter 24. Variations in the working electrode potential relative to reference electrode potential are thus monitored in this case instead of the voltage across the cell.

The embodiments of Figures 3 and 4 can be adapted for monitoring a plurality of cells 10 by connecting the cells in series, with a separate voltmeter 24 across each cell (in the case of Figure 3) or a separate reference electrode 25 and voltmeter 24 for each cell (in the case of Figure 4).

Although voltmeters 21 and 24 have been shown in the drawings, it will be understood that in practice current or voltage measuring chart recorders may be used for monitoring the current or voltage against time. Alternatively, devices for recording the current or voltage values as digital signals may be employed, with a view to further processing of the results.

## Claims

1. A method of detecting micro-organisms in a sample, comprising the steps of placing the sample in a cell so as to be in contact with at least two electrodes, one of which is a working electrode and another a secondary electrode, and applying an electrical DC input across said working and secondary electrodes, characterised by maintaining constant current or constant potential conditions in the cell, and monitoring either potential conditions or current, respectively, in the cell against time.

2. A method according to Claim 1, characterised in that the potential of a third, reference, electrode disposed adjacent to the working electrode is maintained constant with respect to that of the working electrode, and the current flowing through the cell is monitored against time.

3. A method according to Claim 1, characterised in that the potential of the secondary electrode with respect to the working electrode is maintained constant so that the voltage across the cell is kept constant, and the current flowing through the cell is monitored against time.

4. A method according to Claim 1, characterised in that the current flowing in the cell is maintained constant, and the voltage across the cell is monitored against time.

5. A method according to claim 1 characterised in that the current flowing in the cell is maintained constant and the potential of the working electrode with respect to a third, reference, electrode, disposed adjacent to the working electrode, is monitored.

6. A method according to claim 4 or 5 characterised in that a potential difference is established across a fixed resistance between the working electrode and the adjacent side of the DC input and is maintained constant in order to maintain the current constant.

7. A method according to any one of the preceding Claims characterised in that the constant current or potential conditions are established and maintained by means of a potential control amplifier (as herein defined) whose output terminals are connected across the secondary and working electrodes of the cell to form the DC input.

8. A method according to claims 6 and 7 characterised in that the working electrode is connected to the respective output terminal of the potential control amplifier through the fixed resistance and the reference terminal is connected to the same side of the fixed resistance as the working electrode, to maintain the current flowing in the cell constant.

Fig.1.

Fig.2.

Fig.3.

Fig.4.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | PATENT ABSTRACTS OF JAPAN, vol. 10, no. 344 (P-518)[2400], 20th November 1986; & JP-A-61 147 157 (TOSHIBA CORP.) 04-07-1986 * Abstract * | 1-3 | G 01 N 33/48 |
| X | DE-A-3 401 405 (INSTITUT ELECTROCHIMII) * Abstract; figures 2,3 * | 1 | |
| X | US-A-3 506 544 (SILVERMAN et al.) * Column 8, line 57 - column 9, line 66; claim 8; figure 1 * | 1-3 | |
| X | US-A-4 009 078 (WILKINS et al.) * Column 3, lines 10-46 * | 1-5 | |
| A | GB-A-2 029 026 (KYOWA CO.) * Front-page * | 1 | |
| A | FR-A-2 468 648 (K.K. KYOTO DAIICHI KAGAKU) * Whole document * | 1 | TECHNICAL FIELDS SEARCHED (Int. Cl.4) |
| A | US-A-4 321 322 (AHNELL) * Abstract; figure 4 * | 1 | G 01 N |

The present search report has been drawn up for all claims

| Place of search | Date of completion of the search | Examiner |
|---|---|---|
| THE HAGUE | 19-01-1989 | KOUZELIS D. |

EPO FORM 1503 03.82 (P0401)